# EUROPEAN PATENT SPECIFICATION

(11) **EP 0 791 325 B1**
(45) Date of publication and mention of the grant of the patent: **16.03.2005**
(21) Application number: 96109751.6
(22) Date of filing: 18.06.1996
(51) Int. Cl.: A61B 3/032

(54) **Projector of optotypes with variable contrast**
Optotypen-Projektor mit variablem Kontrast
Projecteur d'optotypes avec contraste variable

(30) Priority: 23.02.1996 IT BO960084
(43) Date of publication of application: 27.08.1997
(73) Proprietor: C.S.O. SRL, 50010 Badia a Settimo, Florence (IT)
(72) Inventor: Mura, Sergio, 50018 Scandicci (Florence) (IT)
(74) Representative: Sassatelli, Franco T., Dr.

(56) References cited:
- EP-A- 0 424 792
- GB-A- 1 047 350
- US-A- 5 121 981

## Description

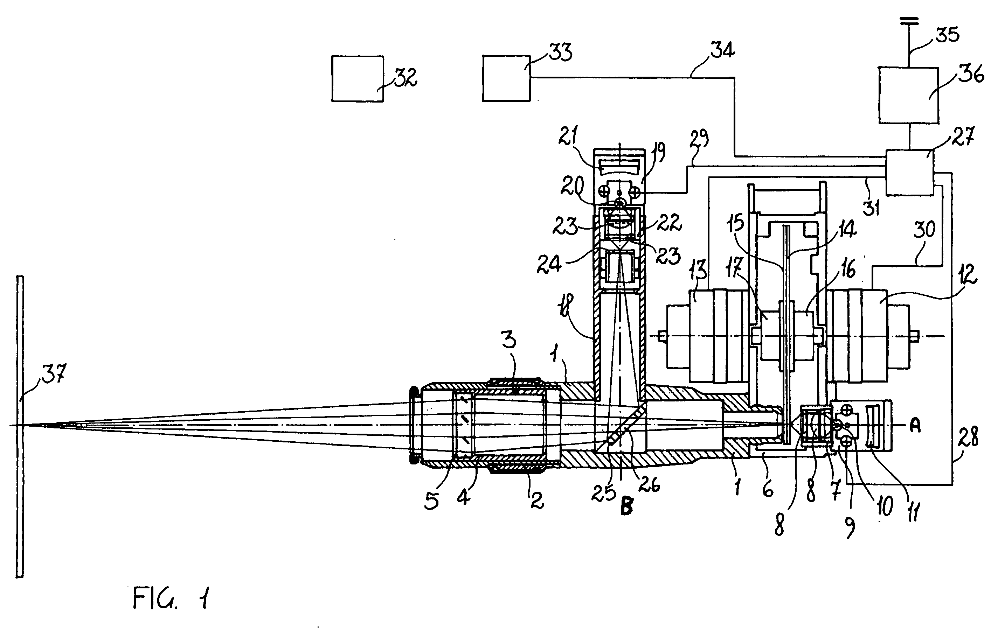

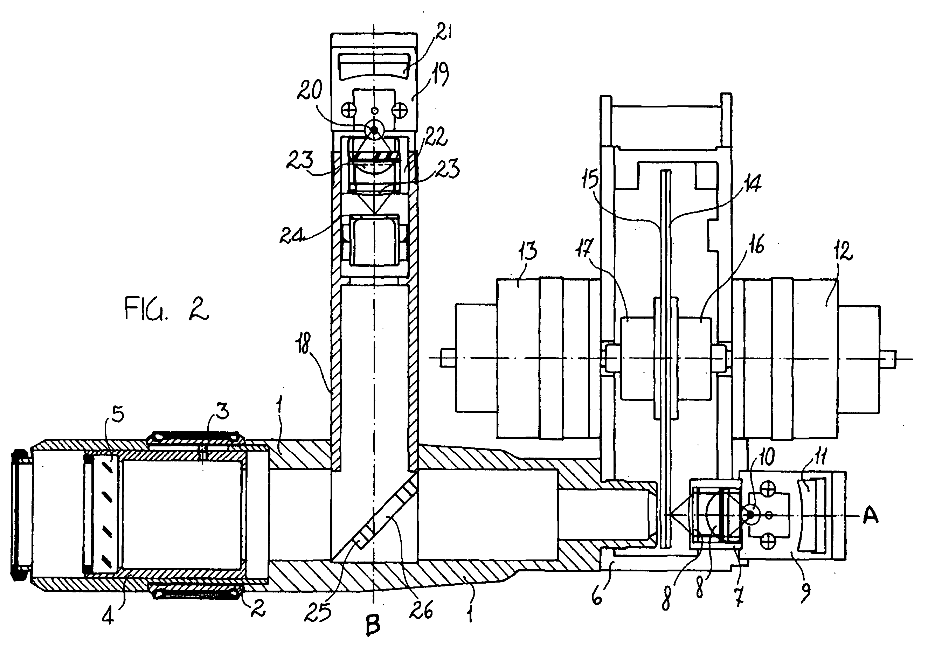

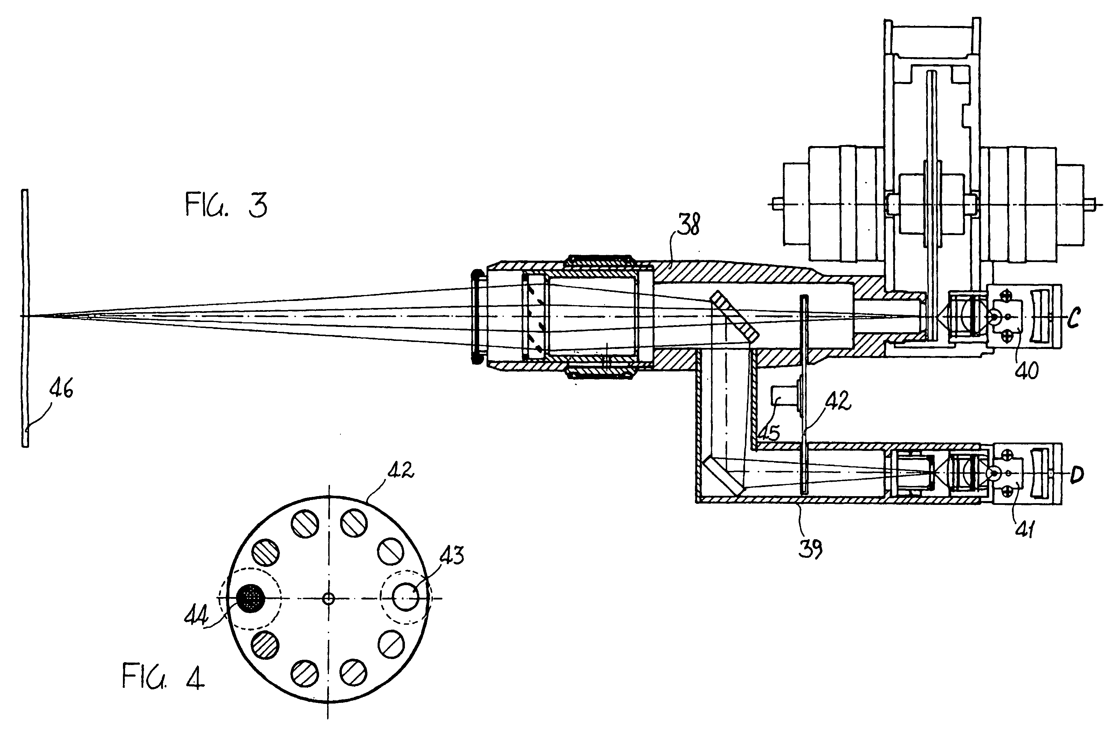

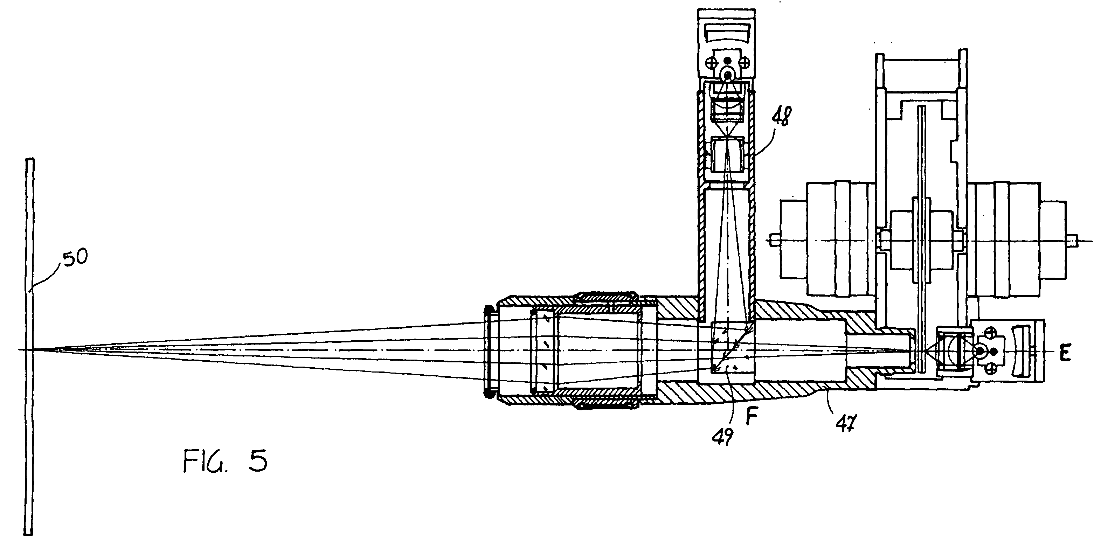

The invention refers to a projector of optotypes which also permits the visual acuity tracking with respect to the varying of the contrast effect. As it is well-known, the visual acuity is affected by the photometric contrast existing between the object and the background; where the photometric cont rast is very high with respect to the difference of the retinal illumination which is produced, even small size objects will be seen. On the contrary, when there is a low contrast effect, even larger objects will not succeed in producing contrasts of such a value to make them perceptible by the retina. At present, the visus examination, also considering the contrast effects, can be made only in intermediate gradualities by means of optotypes printed on tables in translucid plate and illuminated at the back, by varying the light source distance gradually. The large overall dimensions of this equipment, which takes up considerable room in the laboratory, jointed to the need of a wide range of tables, result in slow operative results as, for every graduality and contrasting effect under examination it is necessary to displace the lamp mechanically. Apparatuses are known, as for instance the apparatus described and shown in the US-A-1 047 350. The invented projector goes over the above stated inconveniences as it presents a new equipment which has all the qualities of the projectors of optotypes now in use and in addition it offers new performances such as a best definition of the image, a large variety of optotypes which include special tests for the stereoscopic vision of the chromatic balance, of coincidence and others; moreover, under conditions of extreme accuracy and practical results, given the same ground brightness, it produces the contrast variation of the optotypes image according to the pre-arranged values operating on two complementary light sources by means of a remote control so that by diminishing the intensity of one source the consequent increase of the other one takes place. The projector of optotypes comprises a primary body 1 with a ferrule 2 set on its cylindrical front terminal end, in a possible sliding on a guide 3, for the translation of a first support 4 for focusing the objective 5. Said primary body 1 is brought integral on the other terminal end by a stabilizer 6 which has, on a second support 7, a group of first optical condensers 8 set axially. Said stabilizer 6 holds a first light projector composed of a first lampholder 9, a first lamp 10 and a first concave mirror 11; moreover it features, on an orthogonal prolongation of the frame structure, the motor groups 12 and 13 in a symmetric diagram. The above stated motor groups of which the first motor 12 to allow rotation of a first disk 14 carrying matrix forms and the second motor group 13 to allow rotation of a second disk 15 carrying panels of photoengraved optotypes. Said first and second disks 14 and 15 are countersituated to allow a position reference of said disks during the operative phase. The laying of said disks is determined by a first pinion 16 located between the first motor group 12 and the disk 14, and by another pinion 17 placed between the second motor group 13 and the second disk 15. The primary body 1, in an intermediate orthogonal position, is integral with a secondary body 18 with another light projector at its outer end, such a projector, like the previously described, consists of a second lampholder 19, a second lamp 20 and a second concave mirror 21. On a third support 22 a group of optical condensers 23 is supported to focalize the light of the second lamp 20 on a diagram 24, of the same dimensions of the panel featured on the second disk 15 with engraved optotypes. A mirror 25 with a central hole 26 is positioned at 45° at the intersection point of the first and second axes A and B of the primary and secondary bodies. Lamps 10 and 20 and motor groups 12 and 13 are controlled by a microprocessor 27 on lines 28, 29, 30 and 31. This system can be activated by means of an infrared ray remote control 32, which transmits to a receiver 33 which, on line 34, operates the microprocessor 27 and through the mains supply allowed on line 35 by means of a feeding group 36. In order to make an examination of the visual acuity, also considering the varying of the contrast effect, by means of the remote control 32 the system is activated thus determining the lighting of lamp 10 of the projector housed in the primary body 1 and the positioning by means of the first and second motors 12 and 13 of disks 14 and 15 on the selected optotypes. By means of the concave mirror 11 and of the otptical condensers 8, both the disks 15 and 14 carrying the engraved series of optotypes and the relative matrices which determine the formation of images on the background are lighted up. Then, by making a rotation on the ferrule 2, the objective 5 is focalized thus allowing the formation of the optotype images on the screen 37 under conditions of maximum contrast. Subsequently, by activating the lamp 20 of the projector present in the secondary body 18 by means of the remote control 32, through the group of optical condensers 23, the diagram 24 enlightens and by means both of the mirror 25 and of the objective 5, the light image delimiting the optotypes is projected on screen 37; this image is given by the matrix form present on disk 14, which will move consequently to place itself exactly on the preceding image. As a consequence, by means of the remote control 32 it is possible to operate at the same time on lamps 10 and 20 in a complementary way, so that when the intensity of one lamp diminishes the other one increases as a consequence. Thus obtaining on the screen 37, in presence of the same background brightness, the variation of the control value of the optotype image. In this way, the examination of the visual acuity on different contrast levels is obtained. As an alternative, a projector of optotypes is featured with a rectilinear primary body 38 and a secondary squared up body housing 39 on the axis C of the main projector 40 and on the axis D of a secondary projector 41, a disk carrying a counterposed double series of grey filters with a different absorption gradation between a through hole 43 and a shutter 44. A third group motor 45 operated by a microprocessor by means of a remote control cause the rotation of disk 42 thus obtaining in a sequence, on a screen 46, the image of the optotype with a contrast scalarity maintaining a constant brightness of the background. A further version of this equipment provides a projector of optotypes with a primary body 47 and a secondary body 48 with a glass beam separator 49 positioned at 45°, at the intersection point of the first and second axes E and F of the said primary and secondary bodies 47 and 48, and making the contemporary light transmission and the image reflection at 50% on screen 50. The projector here described and its variants are schematically illustrated in the drawings of sheets 1, 2, 3 and 4. In sheet 1 fig. 1 is a schematic view of the basic projector illustrating both the electronic block working system and the working operations of the image projector set in the primary body 18 which permits, for the complementarity of lamps 10 and 20, the formation of the optotype image on screen 37. In sheet 2 fig. 2 is an enlarged view of the same projector of fig. 1 to better illustrate the two projectors. In sheet 3 fig. 3 is a view of the projector of optotypes carried out in a second version with a rectilinear primary body 38 and a secondary squared up body 39 using a disk 42 for the contemporary transmission of a couple of images of the same optotypes. Fig. 4 is section view of the sole disk 42. In sheet 4 fig. 5 is a view of a third version of the said projector of optotypes, which utilizes a primary body 47 and a secondary body 48 with, at the crossing of their axes E and F, a glass beam separator body 49.

## Claims

1. Projector of optotypes with variable contrast comprising:
- a primary body (1, 38, 47) having a frame structure with a first terminal end, a second terminal end and a first axis (A, E), said primary body being provided on the first terminal end on a cylindrical terminal part thereof with a ferrule (2), a guide (3), a first support (4) and an objective (5) carried by said first support, said ferrule (2) being in a sliding relationship on said guide (3) permitting the translation of the first support (4)for focusing the objective (5);
- a stabilizer (6) brought integrally on the other terminal end of the primary body (1), said stabilizer having a second support (7) supporting a group of first optical condensers (8) set axially and holding a first light projector composed of a first lampholder (9), a first lamp (10) and a first concave mirror (11);
- first and second motor groups (12 and 13) positioned on an orthogonal prolongation of said frame structure in a symmetric diagram, a first disk (14) carrying matrix forms coupled with said first motor group (12) for rotation of said first disk (14) and a second disk (15) carrying panels of photoengraved optotypes coupled with said second motor group (13)for rotation of said second disk (14), said first and second disks (14 and 15) being countersituated to allow a position reference of said disks during an operative phase and where the laying of said disks is determined by a first pinion (16) located between the first motor group (12) and the first disk (14) and by another pinion (17) placed between the second motor group (13) and the second disk (15);
- a secondary body (18,39,48) having a first end, a second end and a second axis (B, F), the first end being integral with said primary body and arranged orthogonally with said primary body in an intermediate position thereof such that the first and the second axes (A and B) of the first and secondary bodies provide an intersection point, said secondary body having, at the second end, a second light projector composed of a second lampholder (19), a second lamp (20), a second concave mirror (21) and a third support (22) supporting a group of second optical condensers (23) to focalize the light of the second lamp (20) on a diagram (24) of the same dimensions of the panel featured on the second disk (15) with engraved optotypes;
- a mirror (25) with a central hole (26) being positioned at 45° at the intersection point of the first and second axes (A and B) of the primary and secondary bodies;
- a remote control (32) capable of determining the lighting of the first lamp (10) of the first projector housed in the primary body and capable of controlling first and second motor groups (12 and 13) for controlling the positioning of first and second disks (14 and 15), in order to make an examination of the visual acuity, also considering the varying of the contrast effect;
- said first concave mirror (11), said first optical condensers (8), said first and second disks (14 and 15 ) together with said first lamp (10) determining the formation of images on a background, and a rotation on the ferrule (2) focusing said objective (5), thus allowing the formation of the optotype images on a screen (37) under conditions of maximum contrast;
- said remote control (32) being adapted to activate the second lamp (20) of the second projector present in the secondary body, through the group of second optical condensers (23), the diagram (24)being enlightened, and by means both of the mirror (25) and of the objective (5) the light image delimiting the optotypes being projected on the screen (37), , in presence of the same background brightness, the variation of the control value of the optotype image and permitting the examination of the visual actuity on different contrast levels.

2. Projector of optotypes with variable contrast, according to claim 1, **characterized in that** it further includes a microprocessor (27) adapted to control the first and second lamps (10 and 20) and the first and second motor groups (12 and 13) in response to said remote control (32), a receiver (33) and a feeding group (36) with a main supply, said receiver (33) and said feeding group (36) being coupled to the microprocessor (27), wherein the remote control (32) is an infrared ray remote control.

3. Projector of optotypes with variable contrast, according to claim 1, **characterized in that** the primary body is a rectilinear primary body (38) having the first light projector (40) aligned with the first axis, the secondary body is a secondary squared up body housing (39) carrying the second light projector (41) on the second axis, the projector further comprising a third disk (42) carrying a counterposed double series of grey filters with a different absorption gradation between a through hole (43) and a shutter (44), a third motor group (45) being operated by a microprocessor (27) in response of the remote control (32) for the rotation of said third disk (42), thus obtaining in a sequence, on the screen (46), the image of the optotype with a contrast scalarity maintaining a constant brightness of the background.

4. Projector of optotypes with variable contrast, according to claim 1, **characterized in that** a glass beam separator (49) is positioned at 45° at the intersection point of the first and second axes (E and F) of the primary body (47) and the secondary body (48), said glass beam separator (49) being capable of making the contemporary light transmission and the image reflection at 50% of the screen (50).

## Patentansprüche

1. Optotypen-Projektor mit variablem Kontrast, umfassend:
- einen ersten Grundkoerper (1, 38, 47), der eine Rahmenstruktur mit einem ersten Anschlussende, einem zweiten Anschlussende und einer ersten Achse' (A, E) aufweist, wobei der erste Grundkoerper an dem ersten Anschlussende auf einem zylindrischen Endabschnitt davon mit einem Klemmring (2), einer Fuehrung (3) einem ersten Traeger (4) und einem Objektiv (5), das von dem ersten Traeger gehalten ist, versehen ist, wobei der Klemmring (2) in einer verschieblichen Beziehung auf der Fuehrung (3) angeordnet ist und die Verschiebung des ersten Traegers (4) zum Fokussieren des Objektivs (5) ermoeglicht;
- eine Stabilisierung (6), die integral auf dem anderen Anschlussende des ersten Grundkoerpers (1) angebracht ist, wobei die Stabilisierung einen zweiten Traeger (7) aufweist, der eine Gruppe von ersten optischen Kondensoren (8) umfasst, die in axialer Richtung eingestellt sind, und der einen ersten Lichtprojektor traegt, der aus einer ersten Lampenhaltung (9), einer ersten Lampe (10) und einem ersten konkaven Spiegel (11) besteht;
- erste und zweite Antriebesgruppen (12 und 13), die auf einer orthogonalen Verlaengerung der genannten Rahmenstruktur in einer symmetrischen Anordnung positioniert sind, wobei eine erste Scheibe (14) Matrixformen traegt, die mit der ersten Antriebsgruppe (12) zur Drehung der ersten Scheibe (14) gekoppelt sind, und wobei eine zweite Scheibe (15) Tafeln mit photomechanisch eingravierten optischen Zeichen traegt, die mit der zweiten Antriebsgruppe (13) zur Drehung der zweiten Scheibe (14) gekoppelt sind, wobei die ersten und zweiten Scheiben (14 und 15) einander gegenueberliegend angeordnet sind, um eine Positionsreferenz der Scheiben waehrend einer operativen Phase zu ermoeglichen, und wobei di Lage der genannten Scheiben durch ein erstes Zahnrad (16), das zwischen der ersten Antriebsgruppe (12) und der ersten Scheibe (14) angeordnet ist, und durch ein weiteres Zahnrad (17), das zwischen der zweiten Antriebsgruppe (13) und der zweiten Scheibe (15) angeordnet ist, festgelegt ist;
- einen zweiten Grundkoerper (18, 39, 48), der ein erstes Ende, ein zweites Ende und eine zweite Achse (B, F) aufweist wobei das erste Ende einteilig mit dem genannten ersten Grundkoerper ausgebildet ist und orthogonal zu dem ersten Grundkoerper in einer Zwischenposition davon angeordnet ist, ' so dass die erste und die zweite Achse (A und B) des ersten und zweiten Grundkoerpers einen Schnittpunkt bilden, wobei der zweite Grundkoerper an dem zweiten Ende einen zweiten Lichtprojektor aufweist, der aus einer zweiten Lampenhalterung (19), einer zweiten Lampe (20), einem zweiten konkaven Spiegel (21) und einem dritten Traeger (22) besteht, der eine Gruppe von zweiten optischen Kondensoren (23) traegt, um das Licht der zweiten Lampe (20) auf ein Diagramm (24) mit den gleichen Abmessungen der auf der zweiten Scheibe (15) vorhandenen Tafel mit eingravierten optischen Zeichen zu fokussieren;
- einen Spiegel (25) mit einer zentralen Oeffnung (26), der unter 45° an dem Schnittpunkt der ersten und zweiten Achse (A und B) der ersten und zweiten Grundkoerpers positioniert ist;
- ein Fernsteuerung (32), die in der Lage ist, das Leuchten der ersten Lampe (10) des ersten Projektors zu bestimmen, der in dem ersten Grundkoerper angeordnet ist, und in der Lage ist, die ersten und zweiten Antriebsgruppen (12 und 13) zu steuern, um die Position der ersten und zweiten Scheiben (14 und 15) zu steuern, um ein Untersuchung der visuellen Schaerfe durchzufuehren, auch unter Betrachtung der Veraenderung der Kontrastwirkung;
- wobei der erste konkave Spiegel (11), die ersten optischen Kondensoren (8), die ersten und zweiten Scheiben (14 und 15) gemeinsam mit der ersten Lampe (10) die Bildung von Bildern auf einem Hintergrund bestimmen, und wobei eine Drehung auf dem Klemmring (2) das Objektiv fokussiert, so dass die Bildung von Bildern der optischen Zeichen auf einem Schirm (37) unter Bedingungen maximalen Kontrasts moeglich ist;
- wobei di Fernsteuerung (32) dazu bestimmt ist, die zweite Lampe (20) des zweiten Projektors, der sich in den zweiten Grundkoerper befindet, zu aktivieren, durch die Gruppe von zweiten optischen Kondensoren (23), wobei das Diagramm (24) beleuchtet ist, und wobei sowohl durch den Spiegel (25) als auch durch das Objektiv di Lichtabbildung, die die optischen Zeichen begrenzt, auf den Schirm (37) projiziert wird, in Anwesenheit der gleichen Hintergrundhelligkeit und der Veraenderung des Kontrollwerts des Bilds des optischen Zeichens, und wobei di Untersuchung der visuellen Schaerfe auf unterschiedlichen Kontrastleveln moeglich ist.

2. Optotypen-Projektor mit variablem Kontrast, gemaess Anspruch 1, **dadurch gekennzeichnet, dass** er ferner einen Mikroprozessor (27) aufweist, der dazu bestimmt ist, die erste und zweite Lampe (10 und 20) und die erste und zweite Antriebsgruppe (12 und 13) entsprechend auf die genannte Fernsteuerung (32) zu steuern, einen Empfaenger (33) und eine Versorgungsgruppe (36) mit einer Hauptzuleitung, wobei der Empfaenger (33) und die Versorgungsgruppe (36) mit dem Mikroprozessor (27) gekoppelt sind, und wobei di Fernsteuerung (32) eine Fernsteuerung mit infraroter Strahlung ist.

3. Optotypen-Projektor mit variablem Kontrast, gemass Anspruch 1, **dadurch gekennzeichnet, dass** der erste Grundkoerper ein geradliniger erste Grundkoerper (38) ist, bei dem der erste Lichtprojektor (40) mit der ersten Achse ausgerichtet ist, wobei der zweite Grundkoerper ein zweites, quadratisches Grundkoerpergehaeuse (39) ist, das den zweiten Lichtprojektor (41) auf der zweiten Achse traegt, wobei der Projektor fernen eine dritte Scheibe (42) aufweist, der gegenueberliegende, doppelte Reihen von Graufiltern mit unterschiedlichen abgestuften Absorptionswerten zwischen einem durchgehenden Loch (43) und einem Verschluss (44) aufweist, wobei eine dritte Antriebsgruppe (45) durch einen Mikroprozessor (27) ansprechend auf die Fernsteuerung (32) fuer die Drehung der dritten Scheibe (42) betaetigt wird, so dass auf dem Schirm (46) das Bild des optischen Zeichens der Reihe nach mit einem abgestuften Kontrast erhalten wird, waehrend eine konstante Helligkeit des Hintergrunds aufrecht erhalten wird.

4. Optotypen-Projektor mit variablem Kontrast, gemaess Anspruch 1, **dadurch gekennzeichnet, dass** ein Strahlenseparator (49) aus Glas unter 45° an einem Schnittpunkt der ersten und Zweiten Achse (E und F) des ersten Grundkoerpers (47) und des zweiten Grundkoerpers (48) angeordnet ist, wobei der Strahlenseparator (49) aus Glas in der Lage ist, die Transmission von Licht und die Reflexion des Bildes gleichzeitig zu 50% der Werts auf den Schirm (50) zu bringen.

## Revendications

1. Projecteur d'optotypes avec contraste variable, qui comprend :
un corps principal (1,38, 47) qui présente une structure de bâti dotée , d'une première extrémité terminale, d'une deuxième extrémité terminale et d'un premier axe (A, E), ledit corps principal étant doté sur la première extrémité terminale de sa partie terminale cylindrique d'un manchon (2), d'un guide (3), d'un premier support (4) et d'un objectif (5) soutenu par ledit premier support, ledit manchon (2) étant disposé à coulissement sur ledit guide (3), ce qui permet le déplacement du premier support (4) pour mettre au point l'objectif (5),
un stabilisateur (6) formé d'un seul tenant sur l'autre extrémité terminale du corps principal (1), ledit stabilisateur présentant un deuxième support (7) qui soutient un groupe de premiers condenseurs optiques (8) qui sont réglés axialement et qui soutiennent un premier projecteur d'éclairage constitué d'un premier support (9) de lampe, d'une première lampe (10) et d'un premier miroir concave (11),
un premier groupe et un deuxième groupe (12 et 13) de moteurs positionnés sur un prolongement orthogonal de ladite structure de bâti dans un diagramme symétrique, un premier disque (14) soutenant des formes de matrice accouplées audit premier groupe (12) de moteurs pour faire tourner ledit premier disque (14) et un deuxième disque (15) soutenant des panneaux d'optotypes photogravés accouplés audit deuxième groupe (13) de moteurs pour faire tourner ledit deuxième disque (15), ledit premier disque et ledit deuxième disque (14 et 15) étant situés l'un contre l'autre pour permettre une référence de position desdits disques pendant une phase d'exécution, la pose desdits disques étant déterminées par un premier pignon (16) situé entre le premier groupe (12) de moteurs et le premier disque (14) et par un autre pignon (17) situé entre la deuxième groupe (13) de moteurs et le deuxième disque (15),
un corps secondaire (18, 39, 48) qui présente une première extrémité, une deuxième extrémité et un deuxième axe (B, F), la première extrémité étant formée d'un seul tenant avec ledit corps principal et étant agencée orthogonalement par rapport audit corps principal lorsque ce dernier se trouve dans une position intermédiaire, de telle sorte que le premier axe et le deuxième axe (A et B) du corps principal et du corps secondaire forment un point d'intersection, ledit corps secondaire présentant sur la deuxième extrémité un deuxième projecteur d'éclairage constitué d'un deuxième support (199 de lampe, d'une deuxième lampe (20), d'un deuxième miroir concave (21) et d'un troisième support (22) qui soutient un groupe de deuxièmes condenseurs optiques (23) pour focaliser la lumière de la deuxième lampe (20) sur un diagramme (24) qui présente les mêmes dimensions que le panneau présent sur le deuxième disque (15) doté d'optotypes gravés,
un miroir (25) doté d'un orifice central (26) qui est positionné a 45° au point d'intersection entre le premier axe et le deuxième axe (A et B) du corps principal et du corps secondaire,
une commande à distance (32) apte à déterminer l'éclairage de la première lampe (10) du premier projecteur enveloppé dans le corps principal et apte à commander le premier groupe et le deuxième groupe (12 et 13) de moteurs pur commander le positionnement du premier disque et du deuxième disque (14 et 15) de manière à réaliser un examen de l'acuité visuelle en tenat également compte de la variation de l'effet de contraste,
ledit premier miroir concave (11), lesdits premiers condenseurs optiques (8), ledit premier disque et ledit deuxième disque (14 et 15) avec ladite première lampe (10) déterminant la formation d'images sur un arrière-plan et une rotation du manchon (2) mettant au point ledit objectif (5), ce qui permet la formation des images d'optotypes sur un écran (37) dans des conditions de contraste maximal,
ladite commande à distance (32) étant adaptée pur activer la deuxième lampe (20) du deuxième projecteur présent dans le corps secondaire par l'intermédiaire du groupe de deuxièmes condenseurs optiques (23), le diagramme (24) étant éclairé et, au moyen tant du miroir (25) que de l'objectif (5), l'image lumineuse qui délimite les optotypes étant projetée sur l'écran (37) en présence de la même luminosité d'arrière-plan, la variation de la valeur de commande de l'image d'optotypes et permettant l'examen de l'acuité visuelle à différents niveaux de contraste.

2. Projecteur d'optotypes avec contraste variable selon la revendication 1, **caractérisé en ce qu'**il comprend en outre un microprocesseur (27) adapté pour commander la première lampe, la deuxième lampe (10, 20), le premier groupe et le deuxième groupe (12 et 13) de moteurs en résponse à ladite commande à distance (32), un récepteur (33) et un groupe d'alimentation (36) doté d'une alimentation principale, ledit récepteur (33) et ledit groupe d'alimentation (36) étant raccordés au microprocesseur (27), la commande à distance (32) étant une commande à distance à rayons infrarouges.

3. Projecteur d'optotypes avec contraste variable selon la revendication 1, **caractérisé en ce que** le corps principal est un corps principal rectiligne (38) dont le premier projecteur d'éclairage (40) est aligné sur le premier axe, le corps secondaire étant un boîtier secondaire (39) à corps carré qui porte le deuxième projecteur d'éclairage (41) sur le deuxième axe, le projecteur comprenant en outre un troisième disque (42) qui porte une double série de filtres gris dotés de différentes gradations d'absorption et situés les uns contre les autre entre un trou de passage (43) et un dispositif de fermeture (44), un troisième groupe (45) de moteurs étant actionné par un microprocesseur (27) en réponse à la commande à distance (32) pour faire tourner ledit troisième disque (42), ce qui permet d'obtenir successivement sur l'écran (46) l'image d'optotype avec une grandeur scalaire de contraste qui maintient un arrière-plan de luminosité constante.

4. Prolecteur d'optotypes avec contraste variable selon la revendication 1, **caractérisé en ce qu'**un séparateur de faisceau (49) en verre est positionné à 45° au point d'intersection entre la premier axe et le deuxième axe (E et F) du corps principal (47) et du corps secondaire (48), ledit séparateur de faisceau (49) en verre étant apte à réaliser simultanément la transmission de la lumière et la réflexion d'image sur 50% de l'écran (50).
